# NEUE EUROPÄISCHE PATENTSCHRIFT

(11) **EP 0 983 752 B2**
(45) Veröffentlichungstag und Bekanntmachung des Hinweises auf die Entscheidung über den Einspruch: **09.06.2010**
(45) Hinweis auf die Patenterteilung: 31.03.2004
(21) Anmeldenummer: 99112417.3
(22) Anmeldetag: 29.06.1999
(51) Int. Cl.: A61F 2/06, A61M 29/00

(54) **Stent**
Stent
Stent

(30) Priorität: 31.08.1998 DE 19839646
(43) Veröffentlichungstag der Anmeldung: 08.03.2000
(73) Patentinhaber: Abbott Laboratories Vascular Enterprises Limited, Dublin 2 (IE)
(72) Erfinder: Oepen, Randolf, von, Dr.-Ing., 72145 Hirrlingen (DE)
(74) Vertreter: Schmitz, Hans-Werner

(56) Entgegenhaltungen:
- EP-A1- 0 830 853
- EP-B1- 0 878 173
- DE-A1- 3 811 231
- DE-A1- 19 537 872
- DE-U1- 29 708 879
- US- - 4 856 516
- US-A- 5 728 104
- & JP 02277467A (Patent abstracts of Japan) + englische Übersetzung
- "Elective Placement of covered Stents in native coronary Arteries" Acta Radiologica 44 (2003), 294-3d, ISSN 0284-1851

## Beschreibung

Die vorliegende Erfindung betrifft einen Stent mit einem Stentkörper, welcher insbesondere zur Aufweitung verengter Hohlgefäße verwendet wird, gemäss dem Oberbegriff des Anspruches 1. Ein derartiger Stent ist aus der DE 195 37 872 A1 bekannt.

Aus dem Stand der Technik sind unterschiedliche Ausgestaltungsformen von Stents bekannt. Diese bilden eine Gefäßprothese, welche aus körperverträglichem Material besteht. Stents werden im allgemeinen dazu verwendet, Hohlgefäße wie z. B. Blutgefäße oder Körperöffnungen aufzuweiten und in einem aufgeweiteten Zustand zu halten. Zu diesem Zwecke wird der Stent normalerweise in einem nicht expandierten Zustand im Körper des Patienten in ein verengtes Hohlgefäß positioniert und nachfolgend durch geeignete Mittel wie beispielsweise einen Ballonkatheter aufgeweitet. Üblicherweise besteht der Stentkörper aus einer Stegstruktur, wobei die Stegstruktur mehrere zueinander benachbarte, jeweils durch Stege begrenzte Zellen aufweist. Beim Expandieren werden die einzelnen Stegbereiche des Stents verformt, so daß dieser dauerhaft in dieser expandierten Form verbleibt.

Ein ähnlicher Stent wird in US-A-5 728 104 gezeigt.

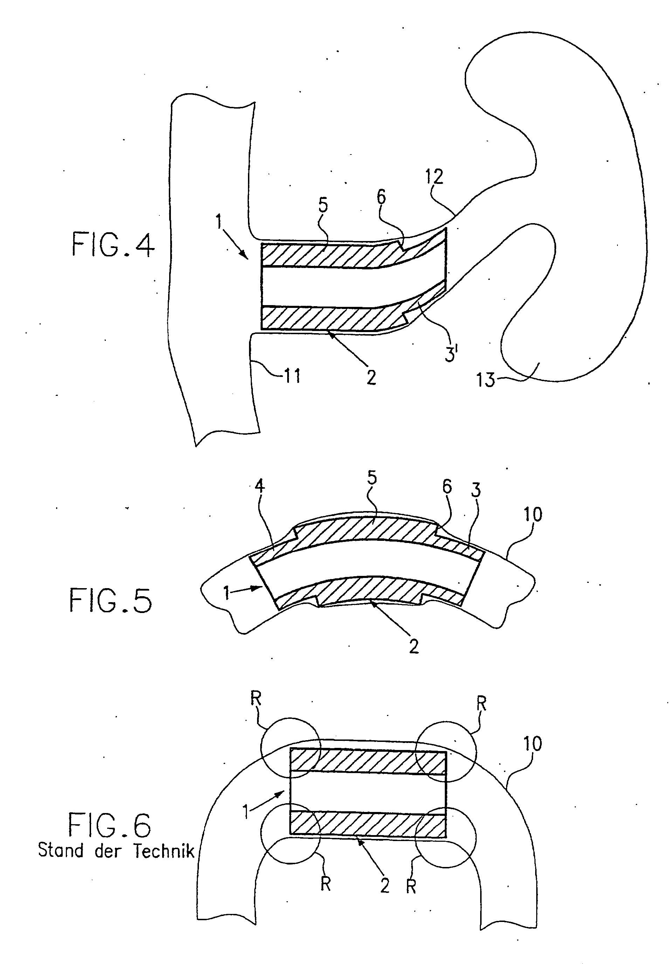

Vielfach tritt bei derart aufgeweiteten Hohlgefäßen das Problem der Restenose auf. Hierbei kommt es nach einiger Zeit zu einer Wiederverengung des durch den Stent aufgeweiteten Bereichs des Hohlgefäßes. Eine derartige Restenose kann u. a. durch die Eigensteifigkeit des Stents hervorgerufen werden. Wie schematisch in Fig. 6 dargestellt, wird das Hohlgefäß 10 durch den Stent 1 versteift. Hierbei kann es insbesondere an den beiden Endbereichen des Stents infolge der Eigensteifigkeit des Stentkörpers 2 zu starken Reizungen R der Gefäßwand kommen, welche zu einer Restenose des Hohlgefäßes führen.

Der Erfindung liegt die Aufgabe zugrunde, einen Stent zu schaffen, welcher die Gefahr einer Restenose eines Hohlgefäßes im Bereich eines implantierten Stents verringert.

Diese Aufgabe wird durch einen Stent mit den Merkmalen des Anspruchs 1 gelöst. Vorteilhafte Weiterbildungen sind Gegenstand der Unteransprüche.

Erfindungsgemäß ist somit ein Stent vorgesehen, bei welchem sich eine Wandstärke des Stents ändert. Hierdurch wird ein Stent mit Bereichen geschaffen, welche infolge der unterschiedlichen Wandstärke verschiedene Eigensteifigkeiten aufweisen. Dadurch weist der Stentkörper in den Bereichen mit geringerer Wandstärke eine höhere Flexibilität auf, wodurch eine Reizung der Gefäßwand an diesen Stellen vermindert bzw. vollständig verhindert werden kann. Somit kann die Gefahr einer Restenose in diesen Bereichen deutlich verringert werden.

Vorzugsweise ist die Wandstärke der beiden Endbereiche des Stentkörpers kleiner als die Wandstärke eines Hauptbereichs des Stentkörpers. Dadurch kann insbesondere die Eigensteifigkeit der beiden Endbereiche des Stentkörpers verringert werden, so daß die Endbereiche eine größere Flexibilität aufweisen und somit eine abrupte "Richtungsänderung" bzw. ein Abknicken des Hohlgefäßes infolge. eines Steifigkeitsgefälles zwischen dem Bereich des Hohlgefäßes ohne Stent und dem Bereich des Hohlgefäßes mit Stent verhindert werden kann. Dadurch ergibt sich ein weicherer Übergang zwischen dem Hohlgefäß und dem Stent, wodurch eine starke Reizung an den Endbereichen des Stents vermieden werden kann.

Gemäß einer bevorzugten Ausgestaltung der vorliegenden Erfindung, weist der Stentkörper genau einen Endbereich auf, welcher eine geringere Wandstärke als der restliche Bereich des Stentkörpers aufweist. Ein derartiger Stent kann vorzugsweise bei Abzweigungen bzw. Nebenästen von Hohlgefäßen verwendet werden. Dieser Stent wird derart in der Abzweigung angeordnet, daß der Endbereich mit geringerer Wandstärke von der Abzweigung fortweist. Somit ist im Bereich der Abzweigung bzw. direkt an der Abzweigung der Stentbereich mit einer größeren Wandstärke angeordnet, da aufgrund des natürlichen Verlaufs der Abzweigung in diesem Bereich die Gefahr einer Reizung infolge des Stents gering ist. In dem von der Abzweigung weiter entfernten Bereich weist der Stent jedoch eine geringere Wandstärke auf, so daß hier eine größere Flexibilität des Stents vorhanden ist und der Stent dem natürlichen Verlauf des Hohlgefäßes folgen kann. Somit ist die Gefahr einer Reizung infolge der hohen Flexibilität des Endbereichs des Stents verringert.

Um einen definierten Übergang zwischen dem Hauptbereich und dem Endbereich mit geringerer Wandstärke zu erreichen, ist der Übergang zwischen diesen beiden Bereichen stufenförmig bzw. abgestuft ausgestaltet. Dadurch kann die Steifigkeit des Stents sprunghaft geändert werden und die gewünschte Flexibilität des Stents in definierten Bereichen des Stentkörpers erhalten werden.

Um eine allmähliche Änderung der Steifigkeit bzw. Flexibilität des Stentkörpers zu erreichen, ist der Übergang zwischen dem Bereich mit größerer Wandstärke zu dem Bereich mit geringerer Wandstärke kontinuierlich verlaufend ausgestaltet. Hierbei kann der Übergang zwischen den beiden Wandstärken beispielsweise linear gestaltet sein oder auch eine sich stetig ändernde Steigerung aufweisen.

Um verschiedene Eigensteifigkeiten der beiden Endbereiche des Stentkörpers bereitzustellen, können die Wandstärken der beiden Endbereiche unterschiedlich sein. Um einen symmetrischen Stent bereitzustellen, können die Endbereiche des Stentkörpers jedoch auch gleiche Wandstärken aufweisen. Weiterhin kann die Flexibilität der Endbereiche auch durch gleiche bzw. unterschiedliche Längen der Endbereiche in Axialrichtung bzw. bei sich allmählich ändernden Wandstärken im Übergangsbereich durch unterschiedliche Axiallängen der Übergangsbereiche erreicht werden.

Nachfolgend wird die Erfindung anhand von Ausführungsbeispielen in Verbindung mit der Zeichnung beschrieben. In der Zeichnung ist:
- Fig. 1: eine schematische Schnittansicht in Längsrichtung eines Stents gemäß einem ersten Ausführungsbeispiel der vorliegenden Erfindung;
- Fig. 2: eine schematische Schnittansicht in Längsrichtung gemäß einem zweiten Ausführungsbeispiel der vorliegenden Erfindung;
- Fig. 3: eine schematische Schnittansicht eines Stents gemäß einem dritten Ausführungsbeispiel der vorliegenden Erfindung;
- Fig. 4: eine schematische Ansicht einer Anordnung des Stents gemäß dem dritten Ausführungsbeispiel der vorliegenden Erfindung in einem Astbereich eines Hohlgefäßes;
- Fig. 5: eine schematische Ansicht der Anordnung eines Stents gemäß dem ersten Ausführungsbeispiel in einem Hohlgefäß;
- Fig. 6: eine schematische Darstellung eines Stents gemäß dem Stand der Technik in einem Hohlgefäß.

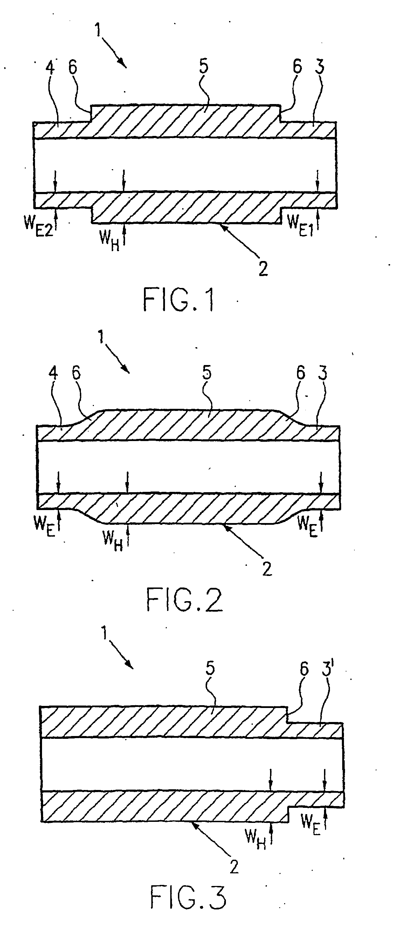

Nachfolgend wird unter Bezugnahme auf Fig. 1 und Fig. 5 ein erstes Ausführungsbeispiel eines erfindungsgemäßen Stents beschrieben. Der Stent 1 besteht aus einem im wesentlichen zylinderförmigen Stentkörper, welcher in seinem Inneren hohl ausgebildet ist, um einen Fluiddurchgang zu ermöglichen. Der Stentkörper 2 besteht aus einer flexiblen Stegstruktur, welche aus Gründen besserer Übersichtlichkeit nicht dargestellt ist. Der Stentkörper 2 des ersten Ausgangsbeispiels ist in drei Bereiche unterteilt, nämlich einen Hauptbereich 5, einen Endbereich 3 sowie einen Endbereich 4 (vgl. Fig. 1). Hierbei ist die Wandstärke W_{E} der Endbereiche 3, 4 im Vergleich zur Wandstärke W_{H} des Hauptbereichs 5 geringer. Im vorliegenden Ausführungsbeispiel beträgt hierbei die Wandstärke W_{E1}, W_{E2} der Endbereiche ungefähr die Hälfte der Wandstärke W_{H} des Hauptbereichs 5. Es sind jedoch beliebige Wandstärkenverhältnisse W_{E}/W_{H} zwischen End- und Hauptbereich möglich. Auch können die Wandstärken W_{E1}, W_{E2} der beiden Endbereiche 3, 4 jeweils unterschiedlich gewählt werden. Ebenfalls kann die Flexibilität des Stents über unterschiedliche Längen der Endbereiche 3, 4 mit geringerer Wandstärke W_{E} beeinflußt werden.

Zwischen dem Hauptbereich 5 und den Endbereichen 3, 4 ist ein Übergang bzw. Übergangsbereich 6 angeordnet, welcher den Hauptbereich mit den jeweiligen Endbereichen 3, 4 verbindet. Wie in Fig. 1 gezeigt, ist der Übergang 6 vom Hauptbereich 5 zu den Endbereichen 3, 4 stufenförmig bzw. abgestuft ausgebildet. Das heißt, aufgrund der unterschiedlichen Wandstärke von Hauptbereich und Endbereichen ergeben sich unterschiedliche Flexibilitäten der jeweiligen Stentbereiche, so daß sich aufgrund der geringeren Wandstärke W_{E} der Endbereiche in diesem Abschnitt eine größere Flexibilität ergibt. Da der Übergang 6 senkrecht zur Oberfläche der Endbereiche bzw. des Hauptbereichs ausgebildet ist, ergibt sich eine sprunghafte Änderung der Flexibilität des Stents in diesem Bereich.

Wie in Fig. 5 gezeigt, kann sich ein derart gestalteter Stent sehr gut an die jeweiligen Gegebenheiten bzw. den natürlichen Verlauf des Gefäßes anpassen. Wie aus Fig. 5 ebenfalls deutlich wird, können sich die Endbereiche 3, 4 an eine relativ starke Krümmung des Hohlgefäßes 10 anpassen. Folglich ergibt sich auch im Bereich des implantierten Stents ein gleichmäßiger, ohne abrupte Richtungsänderungen verlaufender Gefäßverlauf. Somit können insbesondere die bei herkömmlichen Stents auftretenden starken Reizungen an den Endbereichen des Stents verhindert und folglich die Gefahr von Restenosen deutlich reduziert werden.

Zum Vergleich ist in Fig. 6 ein in einem Hohlgefäß 10 implantierter gewöhnlicher Stent 1 dargestellt. Da der Stentkörper 2 des Stents eine gleichmäßige Wandstärke aufweist, ergeben sich insbesondere bei dem gebogen verlaufenden Hohlgefäß 10 an den Endbereichen des Stents 1 Stellen R, welche eine starke Reizung aufweisen. Diese Reizungen R können für eine Restenose und damit für eine Verengung des Hohlgefäßes 10 verantwortlich sein, so daß im Bereich des implantierten Stents eine sogenannte Restenose auftritt. Durch die erfindungsgemäß flexibel ausgestalteten Endbereiche des Stents 1 können diese Stellen R starker Reizung vermieden werden, da sich der gesamte Stent aufgrund der erhöhten Flexibilität im Endbereich an den natürlichen, gebogenen Verlauf des Hohlgefäßes 10 anpassen kann. Folglich wird insbesondere ein Steifigkeitssprung, welcher zwischen dem Hohlgefäß 10 und dem mit Stent versehenen Bereich des Hohlgefäßes üblicherweise auftritt, durch die flexiblen Enden verringert bzw. abgemildert. Somit können Restenosen aufgrund von durch den implantierten Stent hervorgerufenen Reizungen wirksam reduziert werden.

In Fig. 2 ist ein zweites Ausführungsbeispiel eines erfindungsgemäßen Stents 1 dargestellt. Gleiche Teile dieses Stents sind mit den selben Bezugszeichen wie im ersten Ausführungsbeispiel bezeichnet. Der Stent 1 weist einen rohrförmigen Stentkörper 2 auf, welcher aus einem Hauptbereich 5 sowie zwei Endbereichen 3 und 4 besteht. Die Wandstärke W_{E} der Endbereiche 3, 4 ist ungefähr halb so groß wie die Wandstärke W_{H} des Hauptbereichs 5. Der Hauptbereich 5 ist mit den Endbereichen 3, 4 über jeweils einen Übergang bzw. Übergangsbereich 6 verbunden. Der Übergang 6 ist derart ausgestaltet, daß sich ein kontinuierlich verlaufender Übergang zwischen Hauptbereich und Endbereich ergibt (vgl. Fig. 2). Hierbei nimmt die Wandstärke des Stentkörpers 2 ausgehend von den Endbereichen 3, 4 in Richtung des Hauptbereichs 5.zu. Durch diese allmähliche Zunahme der Wandstärke vom End- zum Hauptbereich ändert sich auch die Flexibilität des Stents 1 in diesem Bereich. D. h. die Flexibilität des Übergangsbereichs 6 verringert sich ausgehend vom Endbereich in Richtung des Hauptbereichs. Es ergibt sich im Unterschied zum ersten Ausführungsbeispiel keine abrupte Änderung der Flexibilität bzw. keine sprunghafte Änderung der Eigensteifigkeit des Stents im Übergangsbereich, sondern die Steifigkeit verändert sich im Übergang 6 langsam und stetig.

Somit weist auch der Stent 1 dieses Ausführungsbeispiels eine größere Flexibilität im Endbereich des Stents 1 auf, so daß eine Restenose aufgrund von Reizungen durch den implantierten Stent wirksam verhindert wird. Zusätzlich kann noch der Übergang 6 zwischen den Endbereichen 3, 4 und dem Hauptbereich 5, je nach den gewünschten Anforderungen, derart gestaltet werden, daß sich die Flexibilität des Übergangs 6 fortschreitend bzw. schrittweise ändert. Hierbei kann der Übergang 6 beliebig gestaltet werden. Z. B. kann ein linearer, ein parabelförmiger oder ein mehrere kleine Stufen aufweisender Übergang 6 vorgesehen werden.

In den Fig. 3 und 4 ist ein drittes Ausführungsbeispiel eines erfindungsgemäßen Stents dargestellt. Gleiche Teile des Stents sind wieder mit gleichen Bezugszeichen wie in den ersten beiden Ausführungsbeispielen bezeichnet. Der Stent 1 dieses Ausführungsbeispiels weist einen rohrförmigen Stentkörper 2 auf, welcher einen Hauptbereich 5 aufweist. Im Gegensatz zu den beiden vorhergehenden Ausführungsbeispielen ist am Stentkörper 2 dieses Ausführungsbeispiels nur ein Endbereich 3' angeordnet, welcher eine geringere Wandstärke W_{E} als der Hauptbereich 5 aufweist. Das andere Ende des Stentkörpers 2 weist die gleiche Wandstärke W_{H} wie der Hauptbereich 5 auf (vgl. Fig. 3). Ein Übergang 6 zwischen dem Hauptbereich 5 und dem Endbereich 3' ist wie im ersten Ausführungsbeispiel stufenförmig ausgeführt. Somit ergibt sich am Übergang 6 eine sprunghafte Änderung der Flexibilität des Stentkörpers 2 aufgrund der unterschiedlichen Wandstärken. Der Übergang 6 kann jedoch in beliebiger Weise ausgeführt werden.

Wie in Fig. 4 gezeigt, wird der Stent 1 des dritten Ausführungsbeispiels in erster Linie an Abzweigungen bzw. Verzweigungen von Hohlgefäßen verwendet. In Fig. 4 ist beispielhaft eine Abzweigung bzw. ein Ast 12 von der Aorta 11 zur Niere 13 dargestellt. Da der Ast 12 im direkten Bereich an der Abzweigung von der Aorta 11 im wesentlichen entlang der Abzweigungsrichtung verläuft und keine Verwindungen, kurvenförmige bzw. gekrümmte Verläufe o.ä. aufweist, kann die Flexibilität des Stentkörpers 2 in diesem Bereich normal ausgeführt sein. Somit kann das Ende des Stentkörpers 2 in diesem Bereich die gleiche Wandstärke wie der Hauptbereich 5 aufweisen (vgl. Fig. 4). Der zur Niere 13 gerichtete andere Endbereich 3' des Stentkörpers 2 weist entsprechend den beiden ersten Ausführungsbeispielen wieder eine geringere Wandstärke W_{E} als der Hauptbereich 5 auf. Somit kann sich der Stent 1 in idealer Weise an den natürlichen Verlauf des Hohlgefäßastes 12 anpassen und Reizungen der Gefäßwand an den jeweiligen Endbereichen des Stents können verringert werden. Da sich infolge der Ein- bzw. Ausatemvorgänge Hohlgefäße wie beispielsweise die Niere bewegen, bewegt sich auch das Gefäß 12 stark, welches die Niere 13 und die Aorta 11 miteinander verbindet. Im Vergleich mit einem herkömmlichen Stent wird durch den erfindungsgemäßen Stent des dritten Ausführungsbeispiels die Flexibilität des Gefäßes somit deutlich erhöht und Reizungen der Gefäßwand verringert. Somit ist die Gefahr der Restenose im Bereich des implantierten Stents deutlich verringert.

Die vorhergehende Beschreibung der Ausführungsbeispiele gemäß der vorliegenden Erfindung dient nur zu illustrativen Zwecken und nicht zum Zwecke der Beschränkung der Erfindung. Im Rahmen der Erfindung sind verschiedene Änderungen und Modifikationen möglich, wie in den Patentansprüchen definiert.

## Patentansprüche

1. Stent (1) mit einem aufweitbaren Stentkörper (2), welcher mindestens zwei unterschiedliche Wandstärken aufweist, wobei der Stentkörper (2) wenigstens einen Endbereich (3, 4) aufweist, dessen Wandstärke (W_{E}) kleiner als eine Wandstärke (W_{H}) eines Hauptbereichs (5) ist, **dadurch gekennzeichnet, dass** der Hauptbereich (5) eine konstante Wandstärke (W_{H}) aufweist und der Endbereich (3, 4) eine konstante Wandstärke (W_{E}) aufweist.

2. Stent nach Anspruch 1, **dadurch gekennzeichnet, daß** der Stentkörper (2) zwei Endbereiche (3, 4) und einen zwischen diesen angeordneten Hauptbereich (5) aufweist, dessen Wandstärke (W_{H}) größer als die Wandstärke (W_{E}) der Endbereiche (3, 4) ist.

3. Stent (1) nach Anspruch 1, **dadurch gekennzeichnet, daß** der Stentkörper (2) genau einen Endbereich (3') aufweist, dessen Wandstärke (W_{E}) kleiner als die Wandstärke (W_{H}) des Hauptbereichs (2) ist.

4. Stent nach einem der Ansprüche 1 bis 3 **dadurch gekennzeichnet, daß** ein Übergang (6) vom Hauptbereich (5) zum Endbereich (3, 4; 3') stufenförmig ausgestaltet ist.

5. Stent nach einem der Ansprüche 1 bis 3, **dadurch gekennzeichnet, daß** der Übergang (6) kontinuierlich verläuft.

6. Stent nach Anspruch 5, **dadurch gekennzeichnet, daß** der Übergang (6) linear verläuft.

7. Stent nach einem der Ansprüche 1 bis 6, **dadurch gekennzeichnet, daß** die Wandstärke (W_{E1}) des ersten Endbereichs (3) gleich der Wandstärke (W_{E2}) des zweiten Endbereichs (4) ist.

8. Stent nach einem der Ansprüche 1 bis 6, **dadurch gekennzeichnet, daß** die Wandstärke (W_{E1}) des ersten Endbereichs (3) ungleich der Wandstärke (W_{E2}) des zweiten Endbereichs (4) ist.

9. Stent nach einem der Ansprüche 1 bis 8, **dadurch gekennzeichnet, daß** die Länge des ersten Endbereichs (3) gleich der Länge des zweiten Endbereichs (4) ist.

10. Stent nach einem der Ansprüche 1 bis 8, **dadurch gekennzeichnet, daß** die Länge des ersten Endbereichs (3) ungleich der Länge des zweiten Endbereichs (4) ist.

## Claims

1. Stent (1) having an expandable stent body (2) having at least two different wall thicknesses, the stent body (2) comprising at least one end portion (3, 4), the wall thickness (W_{E}) of which is smaller than a wall thickness (W_{H}) of a main portion (5), **characterized in that** the main portion (5) has a constant wall thickness (W_{H}) and the end portion (3, 4) has a constant wall thickness (W_{E}).

2. Stent according to claim 1, **characterized in that** the stent body (2) has two end regions (3, 4) and a main region (5) arranged between the latter, the wall thickness (W_{H}) of the main region (5) being greater than the wall thickness (W_{E}) of the end regions (3, 4).

3. Stent (1) according to claim 1, **characterized in that** the stent body (2) has just one end region (3'), the wall thickness (W_{E}) of which is less than the wall thickness (W_{H}) of the main region (2).

4. Stent according to one of claims 1 to 3, **characterized in that** a transition (6) from the main region (5) to the end region (3, 4; 3') is designed to be step-like.

5. Stent according to one of claims 1 to 3, **characterized in that** the transition (6) runs continuously.

6. Stent according to claim 5, **characterized in that** the transition (6) runs linearly.

7. Stent according to one of claims 1 to 6, **characterized in that** the wall thickness (W_{E1}) of the first end region (3) is equal to the wall thickness (W_{E2}) of the second end region (4).

8. Stent according to one of claims 1 to 6, **characterized in that** the wall thickness (W_{E1}) of the first end region (3) is not equal to the wall thickness (W_{E2}) of the second end region (4).

9. Stent according to one of claims 1 to 8, **characterized in that** the length of the first end region (3) is equal to the length of the second end region (4).

10. Stent according to one of claims 1 to 8, **characterized in that** the length of the first end region (3) is not equal to the length of the second end region (4).

## Revendications

1. Stent (1) avec un corps de stent (2) élargible qui présente au moins deux épaisseurs de paroi différentes, le corps de stent (2) présentant au moins une extrémité (3, 4) dont l'épaisseur de paroi (W_{E}) est plus petite que l'épaisseur de paroi (W_{H}) d'une zone principale (5), **caractérisé en ce que** la zone principale (5) présente une épaisseur de paroi (W_{H}) constante et l'extrémité (3, 4) présente une épaisseur de paroi (W_{E}) constante.

2. Stent selon la revendication 1, **caractérisé en ce que** le corps de stent (2) présente deux extrémités (3, 4) et, disposée entre celles-ci, une zone principale (5) dont l'épaisseur de paroi (W_{H}) est plus importante que l'épaisseur de paroi (W_{E}) des extrémités (3, 4).

3. Stent (1) selon la revendication 1, **caractérisé en ce que** le corps de stent (2) présente précisément une extrémité (3'), dont l'épaisseur de paroi (W_{E}) est plus petite que l'épaisseur de paroi (W_{H}) de la zone principale (2).

4. Stent selon l'une des revendications 1 à 3, **caractérisé en ce qu'**une région de transition (6) allant de la zone principale (5) à l'extrémité (3, 4 ; 3') est conformée en gradins.

5. Stent selon l'une des revendications 1 à 3, **caractérisé en ce que** la région de transition (6) s'étend de manière continue.

6. Stent selon la revendication 5, **caractérisé en ce que** la région de transition (6) s'étend de manière linéaire.

7. Stent selon l'une des revendications 1 à 6, **caractérisé en ce que** l'épaisseur de paroi (W_{E1}) de la première extrémité (3) est égale à l'épaisseur de paroi (W_{E2}) de la seconde extrémité (4).

8. Stent selon l'une des revendications 1 à 6, **caractérisé en ce que** l'épaisseur de paroi (W_{E1}) de la première extrémité (3) est différente de l'épaisseur de paroi (W_{E2}) de la seconde extrémité (4).

9. Stent selon l'une des revendications 1 à 8, **caractérisé en ce que** la longueur de la première extrémité (3) est égale à la longueur de la seconde extrémité (4).

10. Stent selon l'une des revendications 1 à 8, **caractérisé en ce que** la longueur de la première extrémité (3) est différente de la longueur de la seconde extrémité (4).
